# EUROPÄISCHE PATENTSCHRIFT

(11) **EP 1 197 483 B1**
(45) Veröffentlichungstag und Bekanntmachung des Hinweises auf die Patenterteilung: **02.03.2005**
(21) Anmeldenummer: 01122344.3
(22) Anmeldetag: 19.09.2001
(51) Int. Cl.: C07C 45/62

(54) **Verfahren zur katalytischen Reduktion von Alkinverbindungen**
Process for the catalytic reduction of alkyne compounds
Procédé pour la réduction catalitique de composés alcyniques

(30) Priorität: 28.09.2000 DE 10049271
(43) Veröffentlichungstag der Anmeldung: 17.04.2002
(73) Patentinhaber: BASF AKTIENGESELLSCHAFT, 67056 Ludwigshafen (DE)
(72) Erfinder: Grimmer, Johannes, 67061 Ludwigshafen (DE); Müller, Thomas, Dr., 67246 Dirmstein (DE); Ernst, Hansgeorg, Dr., 67346 Speyer (DE)

(56) Entgegenhaltungen:
- SOUTO, M.L.; JOANN, U.; BABAK, B.; KOJI, N.: HELVETICA CHIMICA ACTA, Bd. 83, 2000, Seiten 2617-2628, XP002252070
- BOLAND, W.; SCHROER, N.; SIELER, C.: HELVETICA CHIMICA ACTA, Bd. 70, 1987, XP009016083
- SYNTHETIC COMMUNICATIONS, Bd. 20, Nr. 22, 1990, Seiten 3421-3425, XP009015954

## Beschreibung

Die vorliegende Erfindung betrifft ein neues Verfahren zur Reduktion von Alkinverbindungen, insbesondere betrifft die Erfindung ein Verfahren zur Herstellung von Cyclohexenderivaten, die als Zwischenprodukte für die Herstellung von Carotinoiden geeignet sind.

Eine Vielzahl der in der Literatur beschriebenen technischen Carotinoid-Synthesen, u.a. die Herstellung von Astaxanthin, verläuft über Cyclohexen-Zwischenprodukte, die neben einer oder mehreren C=C-Doppelbindungen auch eine C≡C-Dreifachbindung enthalten. Zur Ausbildung eines konjugierten Doppelbindungssystems muß diese Dreifachbindung in einem separaten Verfahrensschritt partiell reduziert werden.

Dies kann im Rahmen der in DE-A-43 22 277 beschriebenen Astaxanthin-Synthese im Falle des Alkindiols IVa mit Zink/Essigsäure in Methylenchlorid erfolgen.

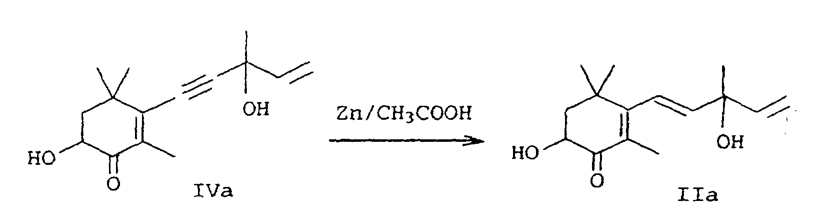

EP-A-0 005 748 betrifft ein weiteres Verfahren zur Herstellung von Astaxanthin, in der die partielle Reduktion des Alkindiols der Formel IIIa ebenfalls mit Zink/Essigsäure in Methylenchlorid durchgeführt wird.

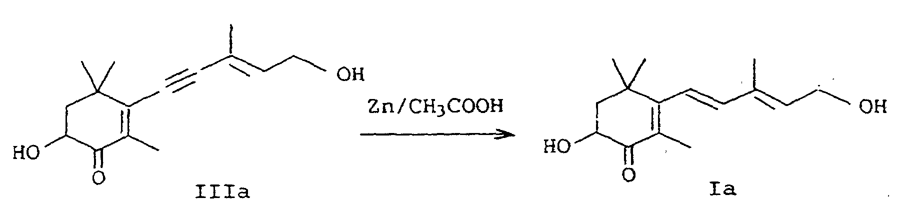

Nachteilig an der beschriebenen Zink/Essigsäure-Reduktion ist die unzureichende Selektivität der Methode. Unerwünschte Nebenprodukte wie z.B. die Bildung von Spiroverbindungen, die sich im weiteren Syntheseverlauf nicht in die gewünschten Folgeprodukte überführen lassen, können zu signifikanten Ausbeuteverlusten führen.

Weitere Reduktionsverfahren sind u.a. in J. Amer. Oil Chem. Soc. 49 (1972) 72 beschrieben, in der die Reduktion von Dreifachbindungen zu cis-Doppelbindungen in langkettigen, konjugierten Fettsäuren mit Zink in siedenden protischen Lösungsmitteln erfolgt.

Die hier genannten drastischen Reduktionsbedingungen sind für thermisch labile Verbindungen nicht geeignet.

In Helv. Chim. Acta 58 (1975) 1016 ist die Reduktion von konjugierten Alkinen in protischen Lösungsmitteln beschrieben. Als Reduktionsmittel verwenden die Autoren Zinkstaub, der durch Zugabe von Kaliumcyanid aktiviert wurde.

Die o.g. Methoden liefern einerseits nur mäßige Ausbeuten, die Aktivierung mit Kaliumcyanid führt andererseits zu einem beträchtlichen Gesundheitsrisiko.

Die Veröffentlichung im Journal für praktische Chemie 336 (1994) 714-715 beinhaltet eine Methode zur (Z)-selektiven Reduktion von konjugierten Dreifachbindungen mit einer Kombination aus Zn (Cu/Ag) in polaren protischen Lösungsmitteln wie z.B. Methanol/Wasser.

Dies Verfahren hat den Nachteil, daß die Herstellung des Reagenzes sehr aufwendig ist und zudem das Reagenz stets frisch hergestellt werden muß.

Es war daher die Aufgabe der vorliegenden Erfindung, ein Verfahren zur partiellen Reduktion von Alkinverbindungen bereitzustellen, mit dem die oben genannten Nachteile des Standes der Technik vermieden werden.

Diese Aufgabe wurde gelöst durch ein Verfahren zur Herstellung von Cyclohexenderivaten der allgemeinen Formeln I oder II, 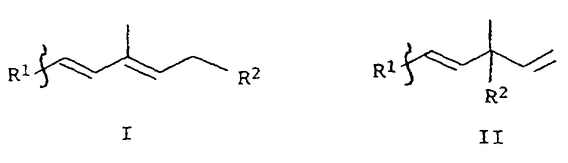 in der die Substituenten R¹ und R² unabhängig voneinander folgende Bedeutung haben:
- R¹: 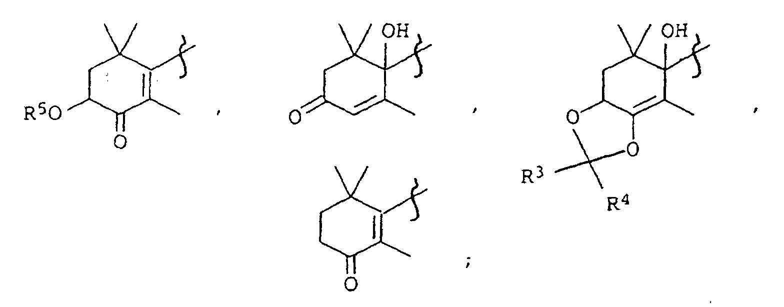
- R²: OH oder eine durch Hydrolyse in eine Hydroxygruppe überführbare Schutzgruppe,
- R³ und R⁴: Wasserstoff, C₁-C₄-Alkyl;
- R⁵: Wasserstoff, C₁-C₄-Acyl;
durch Reduktion von Alkinverbindungen der allgemeinen Formeln III oder IV, 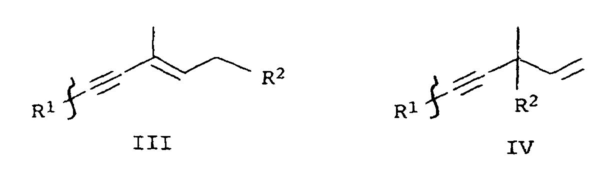 in der die Substituenten R¹ und R² die oben genannte Bedeutung haben, dadurch gekennzeichnet, daß man als Reduktionsmittel ein Gemisch aus Zink und mindestens einer Verbindung B, ausgewählt aus der Gruppe, bestehend aus Ammoniumsalzen, Kupfersalzen, Alkali- und Erdalkalisalzen verwendet.

Als Alkylreste für R³ und R⁴ seien lineare oder verzweigte C₁-C₄-Alkylketten, z.B. Methyl, Ethyl, n-Propyl, 1-Methylethyl, n-Butyl, 1-Methylpropyl-, 2-Methylpropyl sowie 1,1-Dimethylethyl genannt. Bevorzugte Alkylreste sind Methyl und Ethyl.

Die Reste R³ und R⁴ können auch gemeinsam mit dem Kohlenstoffatom, an dem sie gebunden sind, einen Cycloheptyl- oder Cyclohexylring bilden.

Als Substituenten für R⁵ seien lineare oder verzweigte C₁-C₄-Acylketten, z.B. Formyl, Acetyl, Propionyl, Isopropionyl genannt., Bevorzugter Acylrest ist Acetyl.

Unter einer durch Hydrolyse in eine Hydroxygruppe überführbare Schutzgruppe für R² kommen solche funktionellen Gruppen in Betracht, die relativ leicht in die Hydroxygruppe überführt werden können. Genannt seien beispielsweise Ethergruppen, wie

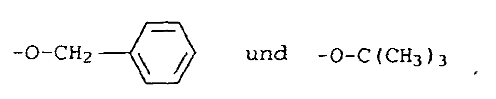

Silylethergruppen, wie -O-Si(CH₃)₃, -O-Si(CH₂CH₃)₃, -O-Si(iso-Propyl)₃, -O-Si(CH₃)₂(tert.-Butyl) und -O-Si(CH₃)₂(n-Hexyl) oder substituierte Methylethergruppen, wie die α-Alkoxy-alkylethergruppen der Formeln 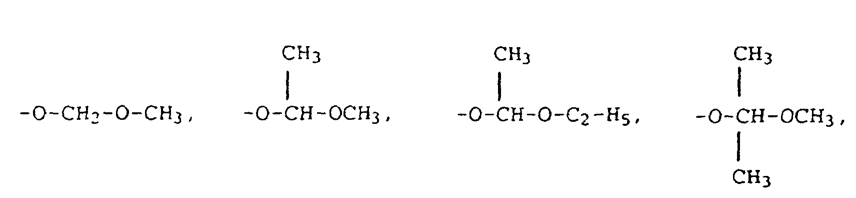 und geeignete Pyranylethergruppen, wie die Tetrahydropyranyloxygruppe und die 4-Methyl-5,6-dihydro-2H-pyranyloxy-Gruppe.

Mit besonderem Vorteil verwendet man für R² die Tetrahydropyranyloxygruppe 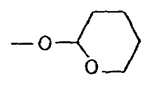 oder die α-Ethoxy-ethoxygruppe der Formel

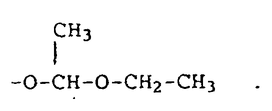

Bedingungen zur Abspaltung der o.g. Schutzgruppen finden sich u.a. in T. Greene "Protective Groups in Organic Chemistry", John Wiley & Sons, 1981, Chapter 2.

Eine bevorzugte Verfahrensvariante ist dadurch gekennzeichnet, daß man als Reduktionsmittel ein Gemisch aus Zink und mindestens einem Ammoniumsalz der Formel V verwendet, 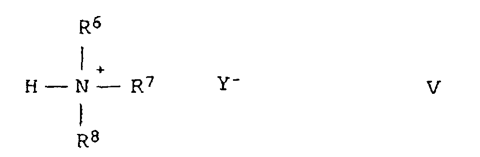 in der die Substituenten unabhängig voneinander folgende Bedeutung haben:
- R⁶ bis R⁸: Wasserstoff, C₁-C₆-Alkyl, Aryl;
- Y⁻: Anion einer organischen oder anorganischen Säure.

Als Alkylreste für R⁶ bis R⁸ seien lineare oder verzweigte C₁-C₆-Alkylketten, z.B. Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl genannt. Bevorzugte Alkylreste sind Methyl, Ethyl, n-Propyl und 1-Methylethyl.

Als besonders bevorzugter Rest für R⁶ bis R⁸ ist Wasserstoff zu nennen.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Für Y⁻ steht generell ein Anion einer organischen oder anorganischen Säure.

Unter organische Säuren sind u.a. aliphatische und aromatische Carbonsäuren, beispielsweise Benzoesäure oder C₁-C₁₂-Alkansäuren, bevorzugt C₁-C₆-Alkansäuren wie Ameisensäure, Essigsäure, Propionsäure, Buttersäure sowie Capronsäure, besonders bevorzugt Essigsäure oder Dicarbonsäuren wie Oxalsäure, Malonsäure bzw. Bernsteinsäure zu verstehen.

Ferner kann Y⁻ auch für Anionen organischer Sulfonsäuren wie Methansulfonat oder *para*-Toluolsulfonat stehen.

Beispiele für anorganische Säuren sind u.a. Chlorwasserstoffsäure, Bromwasserstoffsäure, Kohlensäure, Schwefelsäure, schwefelige Säure, Salpetersäure, salpetrige Säure und Phosphorsäure

Gegenstand der Erfindung ist ebenfalls ein Verfahren zur Herstellung von Cyclohexenderivaten der Formeln I oder II, dadurch gekennzeichnet, daß man als Reduktionsmittel ein Gemisch aus Zink und mindestens einem Kupfersalz, ausgewählt aus der Gruppe, bestehend aus Kupfer(I)bromid, Kupfer(I)chlorid, Kupfer(II)- acetat, Kupfer(II)bromid, Kupfer(II)carbonat, Kupfer(II)chlorid, Kupfer(II)nitrat, Kupfer(II)oxalat und Kupfer(II)sulfat verwendet. Als bevorzugtes Kupfersalz ist Kupfer(II)sulfat zu nennen.

Eine weitere Ausführungsform des erfindungsgemäßen Verfahren ist dadurch gekennzeichnet, daß man als Reduktionsmittel ein Gemisch aus Zink und mindestens einem Alkali- oder Erdalkalisalz, ausgewählt aus der Gruppe, bestehend aus Natriumbromid, Natriumchlorid, Natriumacetat, Natriumcarbonat, Natriumhydrogencarbonat, Natriumoxalat, Natriumsulfat, Kaliumbromid, Kaliumchlorid, Kaliumacetat, Kaliumcarbonat, Kaliumhydrogencarbonat, Kaliumoxalat, Kaliumsulfat sowie die entsprechenden Lithiumsalze, Calciumbromid, Calciumchlorid, Calciumacetat, Calciumcarbonat, Calciumoxalat, Calciumsulfat, Magnesiumbromid, Magnesiumchlorid, Magnesiumacetat, Magnesiumcarbonat, Magnesiumoxalat und Magnesiumsulfat sowie die entsprechenden Bariumsalze verwendet.

Eine besonders bevorzugte Verfahrensvariante ist dadurch gekennzeichnet, daß man als Reduktionsmittel ein Gemisch aus Zink und mindestens einem Ammoniumsalz der Formel V, ausgewählt aus der Gruppe, bestehend aus Ammoniumchlorid, Ammoniumcarbonat, Ammoniumhydrogencarbonat, Ammoniumsulfat und Ammoniumacetat verwendet. Die Substituenten R⁶ bis R⁸ stehen in diesem Fall gemeinsam für Wasserstoff. Als ganz besonders bevorzugtes Ammoniumsalz sei Ammoniumchlorid genannt.

Das erfindungsgemäße Verfahren ist insbesondere zur Herstellung der Cyclohexenverbindungen der Formeln Ia und IIa geeignet.

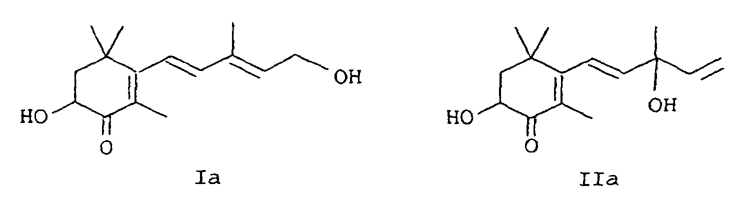

Bei der Durchführung des Verfahrens wird im allgemeinen so gearbeitet, daß man zu den Alkinverbindungen der Formeln III oder IV eine wäßrige Lösung der Verbindung B dosiert und zu dieser Mischung anschließend das Zink zusetzt oder man dosiert eine Suspension aus Zink in der wäßrigen Lösung der Verbindung B zu den o.g. Alkinverbindungen.

Es ist aber auch eine inverse Fahrweise möglich, bei der man das Zink in einer wäßrigen Lösung der Verbindung B suspendiert und zu dieser Suspension die Alkinverbindungen III oder IV gibt.

Es hat sich ferner gezeigt, daß die erfindungsgemäße Reduktion besonders vorteilhaft in Gegenwart von Wasser erfolgt.

Die Wassermenge wählt man so, daß die Verbindung B gelöst oder teilgelöst vorliegt. In der Regel werden pro Mol eingesetztem Zink 15 bis 500 ml Wasser, bevorzugt 20 bis 400 ml, besonders bevorzugt 30 bis 250 ml Wasser verwendet.

Als weiteren Vorteil für den Verlauf der Reduktion hat sich die Zugabe eines inerten Lösungsmittels herausgestellt.

Als inertes Lösungsmittel kommen bei dem erfindungsgemäßen Verfahren generell alle für die Verbindungen I bis IV inerten Solventien in Betracht. Vorzugsweise arbeitet man in chlorierten Kohlenwasserstoffen, wie z.B. in Dichlormethan, Perchlorethylen oder in Chloroform oder in einem etherischen Lösungsmittel, wie Dialkylethern, Tetrahydrofuran oder Dioxan, insbesondere in dem mit Wasser nicht mischbaren Methyl-tert.-butylether. Als weitere Lösungsmittel kommen auch aromatische Kohlenwasserstoffe, insbesondere Toluol sowie C₁-C₃-Alkohole, wie Methanol, Ethanol oder Propanol in Frage.

Vorzugsweise wird eine 10 bis 50 Gew.-%ige Lösung des Alkindiols in einem der o.g. Lösungsmittel, besonders bevorzugt eine 15 bis 30 Gew.-%ige Lösung des Alkindiols in Methylenchlorid verwendet.

Es ist auch möglich, zusätzlich zu den o.g. Lösungsmitteln Essigsäure als Cosolvens einzusetzen.

Bei Anwendung des Zink/Kupfer-Reduktionsmittelsystems fügt man zur Herstellung des Reduktionsmittels pro Mol Zink ca. 0,02 Mol der o.g. Kupfersalze, insbesondere Kupfersulfat in wässriger Lösung hinzu.

Das eingesetzte Zink wird in einer Menge von etwa 0,5 bis 5, bevorzugt 0,7 bis 3, besonders bevorzugt 1 bis 2, ganz besonders bevorzugt 1,2 bis 1,6 Grammatomen pro Mol des zu reduzierenden Alkindiols eingesetzt. Die Dosierung des Zinks kann dabei in einer oder mehreren Portionen erfolgen.

Pro Mol Zink werden 0,5 bis 5 Mol, bevorzugt 0,7 bis 3 Mol, besonders bevorzugt 1 bis 2 Mol der Verbindung B eingesetzt.

Die Reduktion kann bei Temperaturen zwischen 0°C und dem Siedepunkt des entsprechenden Lösungsmittels durchgeführt werden. Bevorzugte Reaktionstemperaturen liegen im Bereich von 10 bis 80°C, besonders bevorzugt im Bereich von 35-45°C.

Anhand der folgenden Beispiele soll der Gegenstand der vorliegenden Erfindung näher erläutert werden.

### Beispiel 1

100 g (0,4 Mol) 6-Hydroxy-3-(3-hydroxy-3-methyl-4-penten-1-inyl)-2,4,4-trimethyl-2-cyclo-hexen-1-on der Formel IVa mit einer Reinheit von 92% wurden in 400 ml Methylenchlorid gelöst und mit einer Lösung von 28,7 g (0,54 Mol) Ammoniumchlorid in 100 ml Wasser vermischt. Das Gemisch wurde auf 10°C abgekühlt, mit 35,2 g (0,54 Mol) Zinkpulver versetzt und ohne zusätzliche Kühlung 2 Stunden gerührt. Anschließend wurde die Reaktionsmischung auf Rückfluß (36-40°C) erwärmt und weitere 3 Stunden bei dieser Temperatur gerührt. Nach Abkühlung auf 10°C wurde abfiltriert und der Rückstand 2 x mit je 100 ml Methylenchlorid gewaschen. Die Mutterlauge und das Waschfiltrat wurden vereinigt und mit 200 ml Wasser ausgeschüttelt. Nach Abdestillation des Lösungsmittels erhielt man einen öligen Rückstand, der nach gaschromatographischer Analyse 78,1 GC-Fl-% an Alkendiol der Formel IIa und 3,3 GC-Fl-% an Alkindiol der Formel IVa enthielt.

### Beispiel 2

Eine Lösung von 100 g (0,4 Mol) Alkindiol der Formel IVa (Reinheit: 92 %ig) in 200 ml Methylenchlorid wurde innerhalb von 2 Stunden bei 25°C unter Rühren zu einer Suspension von 28,7 g (0,54 Mol) Ammoniumchlorid und 35,2 g (0,54 Mol) Zinkpulver in 100 ml Wasser getropft. Nach Ende der Zutropfzeit wurde auf 35°C erwärmt und 18 Stunden bei 35°C gerührt. Gaschromatographisch wurde ein Gehalt von 77,36 GC-Fl-% Alkendiol (Formel IIa) und 12,95 GC-Fl-% Alkindiol (Formel IVa) bestimmt. Nach Zugabe von weiteren 5,74 g (0,11 Mol) Ammoniumchlorid, gelöst in 20 ml Wasser und 7 g (0,11 Mol) Zinkpulver und einer Reaktionszeit von weiteren 2 Stunden bei 35°C ergab die gaschromatographische Analyse einen Gehalt an Alkendiol von 87,9 GC- Fl-% (Formel IIa) und Alkindiol von < 1 GC-Fl-% (Formel IVa).

### Beispiel 3

100 g (0,4 Mol) Alkindiol der Formel IVa (Reinheit: 92 %ig) wurden in 400 ml Methylenchlorid gelöst. Unter Rühren wurden der Reihenfolge nach 28,7 g (0,54 Mol) Ammoniumchlorid und 35,2 g (0,54 Mol) Zinkpulver eingetragen. Bei Raumtemperatur wurden 25 ml Wasser hinzugefügt und unter Rückfluß (38-40°C) 12 Stunden gerührt. Nach Aufarbeitung erhielt man einen Rückstand, der nach gaschromatographischer Analyse 81,2 GC-Fl.-% Alkendiol der Formel IIa und 3,3 GC-Fl.-% Alkindiol der Formel IVa enthielt.

### Beispiel 4

100 g (0,4 Mol) Alkindiol der Formel IVa (92 %ig) wurden in 400 ml Methylenchlorid gelöst und zu 51,8 g (0,54 Mol) Ammoniumcarbonat, gelöst in 100 ml Wasser zugeben. Anschließend wurden 35,2 g (0,54 Mol) Zinkpulver eingetragen. Unter Rühren wurde auf 36-38°C erwärmt. Dabei trat eine heftige CO₂-Entwicklung ein. Nach einer Rührzeit von 12 Stunden bei einer Temperatur von 36-38° wurde eine Probe per GC analysiert: Gehalt Alkendiol: 73,87 GC-Fl.-% und Alkindiol: 4 GC-Fl.-%.

### Beispiel 5

a) Herstellen des Zn/Cu-Reduktionsmittels (gemäß DRP 84891) 100 g Zn-Pulver wurden in 150 ml VE-Wasser suspendiert und unter gutem Rühren bei Raumtemperatur (-25°C) 4 g Kupfersulfat eingetragen. Es wird so lange gerührt, bis in der Suspension die Blaufärbung verschwunden ist. Nach 1 Stunde Nachrührzeit wird abgesaugt und mit VE-Wasser gewaschen. Das feuchte Produkt wurde ausgewogen = 131 g.
b) Reduktion des Alkindiols mit Zn/Cu:
   100 g (0,4 Mol) Alkindiol der Formel IVa wurden in 400 ml Methylenchlorid gelöst. Aus dem unter a) hergestellten feuchten Zn/Cu-Reduktionsmittel wurden 46,3 g (ca. 0,54 Mol) entnommen, in 100 ml Wasser suspendiert und in die Alkindiol/Methylenchlorid-Lösung eingetragen. Nach Zugabe des Reduktionsmittels wurde auf 36°C erwärmt. Nach 6 Stunden Rührzeit wurde eine GC-Analyse durchgeführt: 78,4 GC-Fl.-% Alkendiol (Formel IIa) und 9,7 GC- Fl.-% Alkindiol (Formel IVa).

### Beispiel 6 (Vergleichsbeispiel)

100 g (0,4 Mol) Alkindiol der Formel IVa (92 %ig) wurden in 400 ml Methylenchlorid gelöst. Bei 0°C wurden 80 ml Essigsäure zugegeben und im Abstand von 15 min in 8 Portionen a 4,4 g insgesamt 35,2 g (0,54 Mol) Zinkpulver eingetragen. Nach einer Rührzeit von 45 min bei 0° wurde Zinkacetat abgesaugt und mit Methylenchlorid wertproduktfrei gewaschen. Das Filtrat wurde nach Aufarbeitung per GC analysiert und enthielt 62 GC-Fl-% an Alkendiol der Formel IIa und 21 GC-Fl-% Spiro-Verbindung der Formel VI

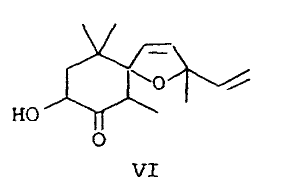

## Patentansprüche

1. Verfahren zur Herstellung von Cyclohexenderivaten der allgemeinen Formeln I oder II, 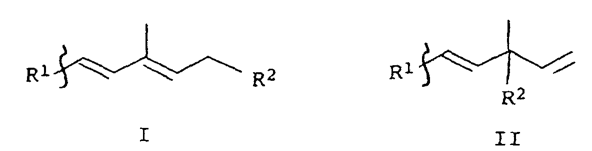 in der die Substituenten R¹ und R² unabhängig voneinander folgende Bedeutung haben:
R¹ 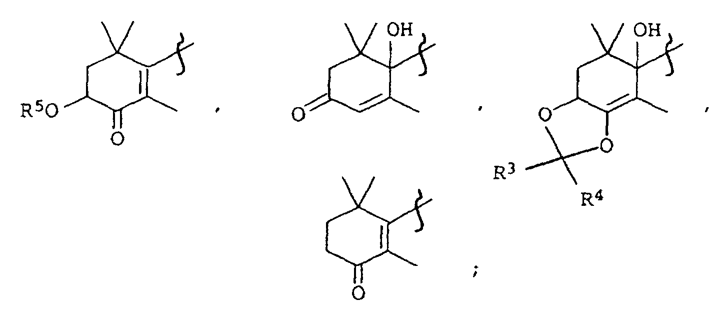
R² OH oder eine durch Hydrolyse in eine Hydroxygruppe überführbare Schutzgruppe,
R³ und R⁴ Wasserstoff, C₁-C₄-Alkyl;
R⁵ Wasserstoff, C₁-C₄-Acyl;
durch Reduktion von Alkinverbindungen der allgemeinen Formeln III oder IV, 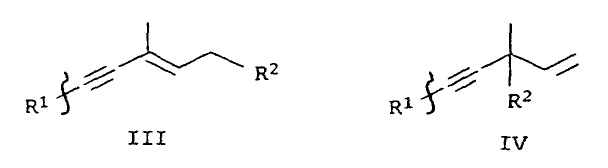 in der die Substituenten R¹ und R² die oben genannte Bedeutung haben, **dadurch gekennzeichnet, daß** man als Reduktionsmittel ein Gemisch aus Zink und mindestens einer Verbindung B, ausgewählt aus der Gruppe, bestehend aus Ammoniumsalzen, Kupfersalzen, Alkali- und Erdalkalisalzen verwendet.

2. Verfahren nach Anspruch 1, **dadurch gekennzeichnet, daß** man als Reduktionsmittel ein Gemisch aus Zink und mindestens einem Ammoniumsalz der Formel V verwendet, 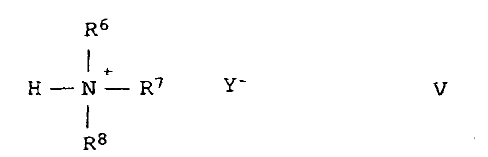 in der die Substituenten unabhängig voneinander folgende Bedeutung haben:
R⁶ bis R⁸ Wasserstoff, C₁-C₆-Alkyl, Aryl;
Y⁻ Anion einer organischen oder anorganischen Säure.

3. Verfahren nach Anspruch 2, **dadurch gekennzeichnet, daß** man als Reduktionsmittel ein Gemisch aus Zink und mindestens einem Ammoniumsalz, ausgewählt aus der Gruppe, bestehend aus Ammoniumchlorid, Ammoniumcarbonat, Ammoniumhydrogencarbonat, Ammoniumsulfat und Ammoniumacetat verwendet.

4. Verfahren nach Anspruch 1, **dadurch gekennzeichnet, daß** man als Reduktionsmittel ein Gemisch aus Zink und mindestens einem Kupfersalz, ausgewählt aus der Gruppe, bestehend aus Kupfer(I)bromid, Kupfer(I)chlorid, Kupfer(II)acetat, Kupfer(II)bromid, Kupfer(II)carbonat, Kupfer(II)chlorid, Kupfer(II)nitrat, Kupfer(II)oxalat und Kupfer(II)sulfat verwendet.

5. Verfahren nach einem der Ansprüche 1 bis 4, **dadurch gekennzeichnet, daß** man die Reduktion in Gegenwart von Wasser durchführt.

6. Verfahren nach einem der Ansprüche 1 bis 5, **dadurch gekennzeichnet, daß** man die Reduktion in einem gegenüber den Cyclohexenderivaten der allgemeinen Formeln I bis IV inerten organischen Lösungsmittel durchführt.

7. Verfahren nach einem der Ansprüche 1 bis 6 zur Herstellung der Cyclohexenverbindungen der Formeln Ia und IIa.

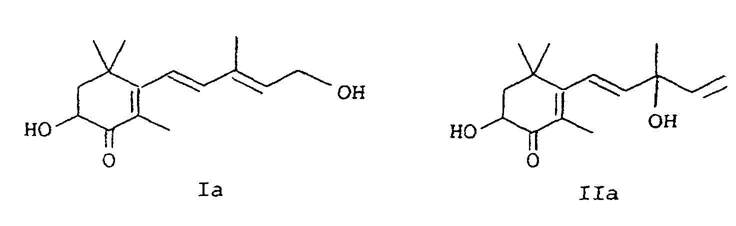

## Claims

1. A process for the preparation of cyclohexene derivatives of general formulae I or II: 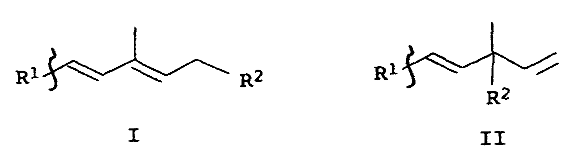 in which the substituents R¹ and R² independently of one another are defined as follows:
R¹ is 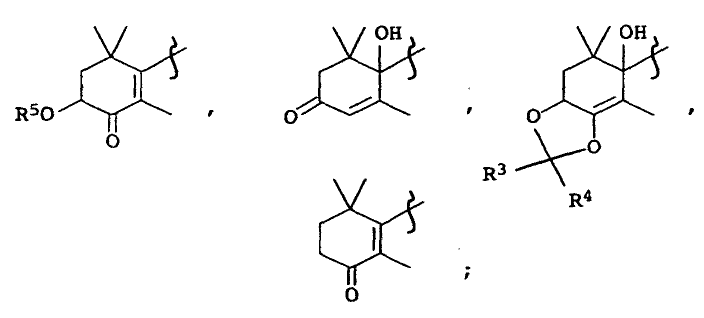
R² is OH or a protective group convertible to a hydroxyl group by hydrolysis;
R³ and R⁴ are hydrogen or C₁-C₄-alkyl; and
R⁵ is hydrogen or C₁-C₄-acyl,
by the reduction of alkyne compounds of general formulae III or IV: 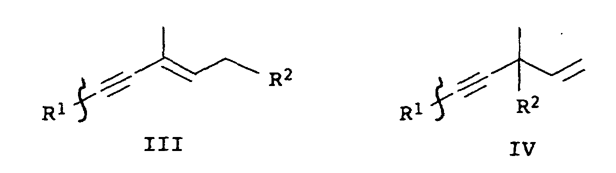 in which the substituents R¹ and R² are as defined above, wherein the reducing agent used is a mixture of zinc and at least one compound B selected from the group consisting of ammonium salts, copper salts and alkali metal and alkaline earth metal salts.

2. A process as claimed in claim 1 wherein the reducing agent used is a mixture of zinc and at least one ammonium salt of formula V: 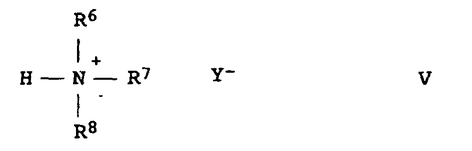 in which the substituents independently of one another are defined as follows:
R⁶ to R⁸ are hydrogen, C₁-C₆-alkyl or aryl; and
Y⁻ is an anion of an organic or inorganic acid.

3. A process as claimed in claim 2 wherein the reducing agent used is a mixture of zinc and at least one ammonium salt selected from the group consisting of ammonium chloride, ammonium carbonate, ammonium hydrogencarbonate, ammonium sulfate and ammonium acetate.

4. A process as claimed in claim 1 wherein the reducing agent used is a mixture of zinc and at least one copper salt selected from the group consisting of copper(I) bromide, copper(I) chloride, copper(II) acetate, copper(II) bromide, copper(II) carbonate, copper(II) chloride, copper(II) nitrate, copper(II) oxalate and copper(II) sulfate.

5. A process as claimed in any of claims 1 to 4 wherein the reduction is carried out in the presence of water.

6. A process as claimed in any of claims 1 to 5 wherein the reduction is carried out in an organic solvent inert toward the cyclohexene derivatives of general formulae I to IV.

7. A process as claimed in any of claims 1 to 6 for the preparation of cyclohexene compounds of formulae Ia and IIa.

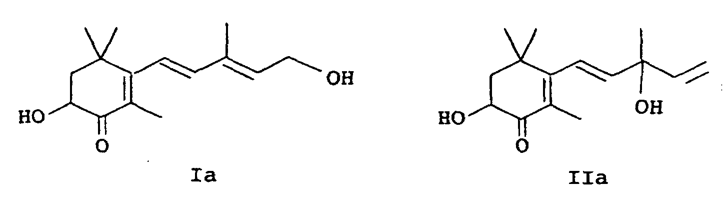

## Revendications

1. Procédé de préparation de dérivés de cyclohexène de formule générale I ou II 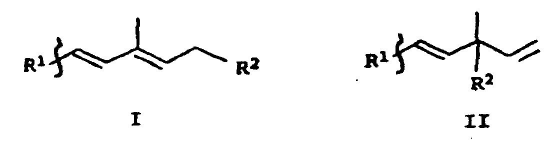 dans laquelle les substituants R¹ et R² ont, indépendamment l'un de l'autre, la signification suivante: 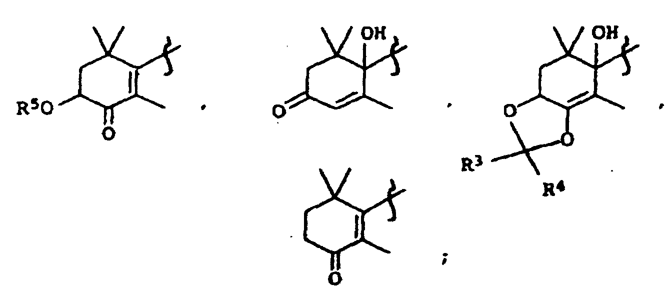
R² désigne OH ou un groupe de protection transformable en un radical hydroxy par hydrolyse;
R³ et R⁴ désignent l'hydrogène ou un radical alkyle C₁-C₄;
R⁵ désigne l'hydrogène ou un radical acyle C₁-C₄;
par réduction de composés alcyniques de formule générale III ou IV 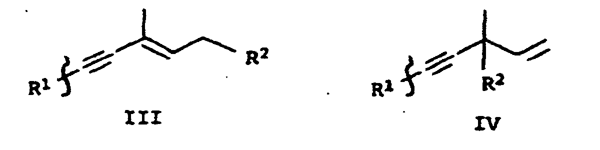 dans laquelle les substituants R¹ et R² ont la signification précitée, **caractérisé en ce qu'**on utilise comme réducteur un mélange de zinc et d'au moins un composé B, sélectionné dans le groupe composé des sels d'ammonium, des sels de cuivre, des sels alcalins et alcalino-terreux.

2. Procédé selon la revendication 1, **caractérisé en ce que** l'on utilise comme réducteur un mélange de zinc et d'au moins un sel d'ammonium de formule V, 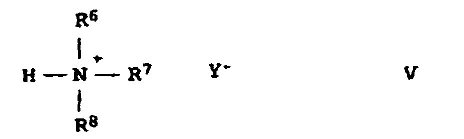 dans laquelle les substituants ont, indépendamment l'un de l'autre, la signification suivante:
R⁶ à R⁸ désignent l'hydrogène, un radical alkyle C₁-C₆ ou aryle;
Y⁻ désigne un anion d'un acide organique ou inorganique.

3. Procédé selon la revendication 2, **caractérisé en ce que** l'on utilise comme réducteur un mélange de zinc et d'au moins un sel d'ammonium sélectionné dans le groupe composé du chlorure d'ammonium, du carbonate d'ammonium, de l'hydrogénocarbonate d'ammonium, du sulfate d'ammonium et de l'acétate d'ammonium.

4. Procédé selon la revendication 1, **caractérisé en ce que** l'on utilise comme réducteur un mélange de zinc et d'au moins un sel de cuivre sélectionné dans le groupe composé du bromure de cuivre (I), du chlorure de cuivre (I), de l'acétate de cuivre (II), du bromure de cuivre (II), du carbonate de cuivre (II), du chlorure de cuivre (II), du nitrate de cuivre (II), de l'oxalate de cuivre (II) et du sulfate de cuivre (II).

5. Procédé selon l'une des revendications 1 à 4, **caractérisé en ce que** l'on procède à la réduction en présence d'eau.

6. Procédé selon l'une des revendications 1 à 5, **caractérisé en ce que** l'on procède à la réduction dans un solvant organique inerte par rapport aux dérivés de cyclohexène de formule générale I à IV.

7. Procédé selon l'une des revendications 1 à 6 pour la préparation des composés de cyclohexène de formule Ia et IIa.
